# EUROPEAN PATENT APPLICATION

(11) **EP 1 837 048 A1**
(43) Date of publication of application: **26.09.2007**
(21) Application number: 07104164.4
(22) Date of filing: 14.03.2007
(51) Int. Cl.: A61M 5/14, A61M 5/168

(54) **Control system for liquid infusion apparatus**

(30) Priority: 20.03.2006 JP 2006076743
(71) Applicant: JAPAN SERVO CO., LTD., Chiyoda-Ku Tokyo (JP); JMS Co., Ltd., Hiroshima-shi (JP)
(72) Inventor: Kato, Hiroshi, Kiryu-shi Gunma (JP); Kawamata, Shunichi, Kiryu-shi Gunma (JP); Kojima, Seiji, Kiryu-shi Gunma (JP); Kagawa, Yoshihisa, Kiryu-shi Gunma (JP); Mizukami, Koji, Kiryu-shi Gunma (JP); Matsumoto, Takahiro, Chiyoda-cho Yamagata-gun Hiroshima (JP)
(74) Representative: Kronthaler, Wolfgang N.K.

(57) **Abstract**

A control system for a liquid infusion apparatus (1) comprises a plurality of liquid infusion devices (1a-1c), each having a common connector (2) of liquid infusion device side for an electric power supply line and a communication line (7), a plurality of racks (3) stacked one another, each for receiving each of the liquid infusion devices, a plurality of common connectors (4) of rack side, each mounted on each of the racks a base unit, and a connector provided on the base unit (6) so as to connect an electric power supply line and a communication line to the connectors of lack side. Each of the common connectors of rack side is connected to each of the common connectors of liquid infusion device side, when each of the liquid infusion devices is inserted into each of the respective racks... A network is used for the communication line, and a warning signal is transmitted from any of the liquid infusion devices through the network to the other liquid infusion devices.

## Description

The present invention relates to a control system for a liquid infusion apparatus, and more particularly, relates to a control system for a liquid infusion apparatus, wherein a liquid medicine etc, is injected into the human body by moving a pusher of syringe by a prime mover.

An apparatus for indicating an abnormal state of the liquid infusion device is disclosed in the Japanese Patent Application Laid-Open No.7626/93.

In case that a plurality of racks, each having a liquid infusion device are used by stacking one another, it is necessary to connect a communication line and an electric power supply line to each of the liquid infusion devices, and such connection works are very complicated.

In case that the warning signal generating function of the liquid infusion device is braked down, no warning signal is outputted, so that the user cannot know the brake down of the warning signal generating function.

Further, in case that the liquid medicine infusion operation of a liquid infusion device is stopped by the warning signal, the stop of the liquid medicine infusion is continued until the user releases the warning state and starts again the liquid infusion operation.

An object of the present invention is to obviate the above defects.

The above object can be accomplished by a control system for a liquid infusion apparatus comprising a plurality of liquid infusion devices, each having a common connector of liquid infusion device side for an electric power supply line and a communication line, a plurality of racks stacked one another, each for receiving each of the liquid infusion devices, a plurality of common connectors of rack side, each mounted on each of the racks so as to be connected to each of the common connectors of liquid infusion device side, when each of the liquid infusion devices is inserted into each of the respective racks, a base unit, and a connector provided on the base unit so as to connect an electric power supply line and a communication line to the common connectors of lack side.

Another object of the present invention is to provide the control system of the liquid infusion apparatus, wherein a network is used for the communication line, and a warning signal is transmitted from any of the liquid infusion devices through the network to the other liquid infusion devices.

Further object of the present invention is to provide the liquid infusion apparatus, wherein when the warning signal is generated in any of the liquid infusion devices in the network and the liquid infusion is stopped, the liquid infusion is carried out by the other liquid infusion device in the network.

These and other aspects and objects of the present invention will be better appreciated and understood when considered in conjunction with the following description and the accompanying drawings. It should be understood, however, that the following description, while indicating preferred embodiments of the present invention, is given by way of illustration and not of limitation. Many changes and modifications may be made within the scope of the present invention without departing from the spirit thereof, and the invention includes all such modifications.

FIG. 1 is a front view of a liquid infusion device according to the present invention.

FIG. 2 is a bottom view of the liquid infusion device shown in FIG. 1.

FIG. 3 is a side view of the liquid infusion device shown in FIG. 1.

FIG.4 is a front view of a liquid infusion apparatus, wherein a plurality of the liquid infusion devices are stacked one another.

FIG. 5 is a side view of the liquid infusion apparatus shown in FIG. 4.

FIG. 6 is an exploded side view of the liquid infusion apparatus shown in FIG. 5.

### DETAILED DESCRIPTION OF THE PREFERRED EMBODIMENT

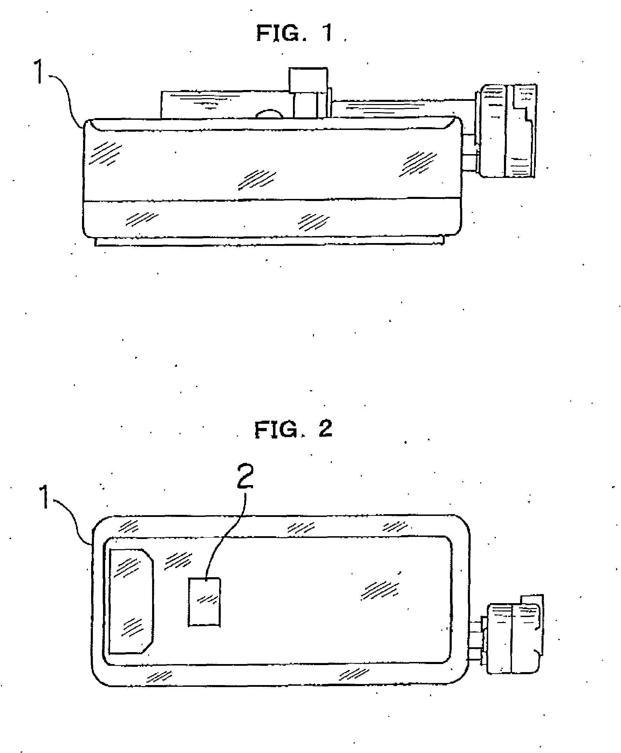

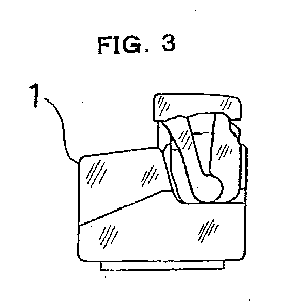

FIG. 1 to FIG. 3 show a liquid infusion device 1.

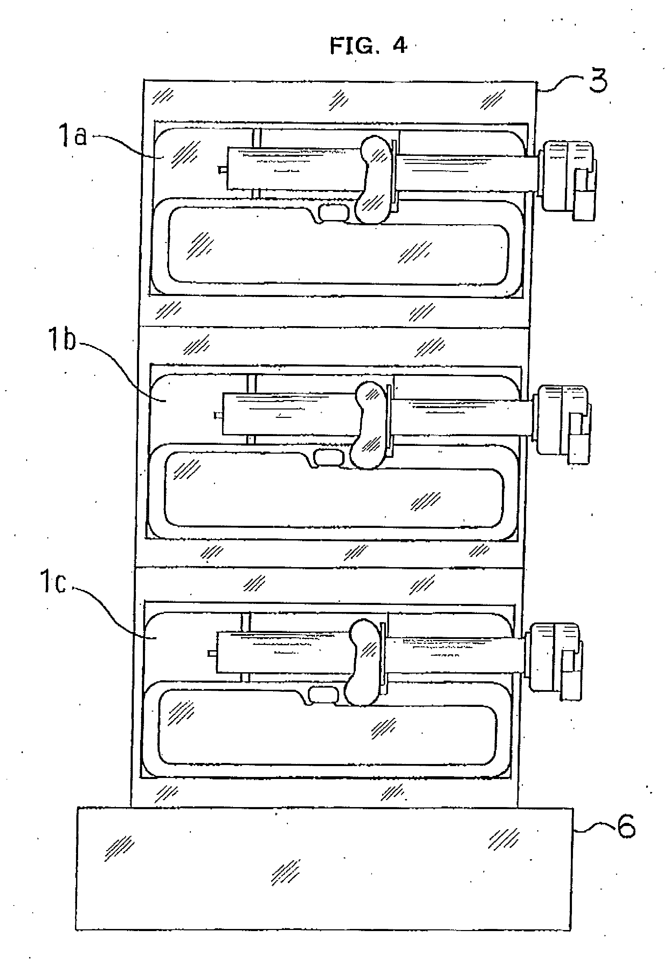

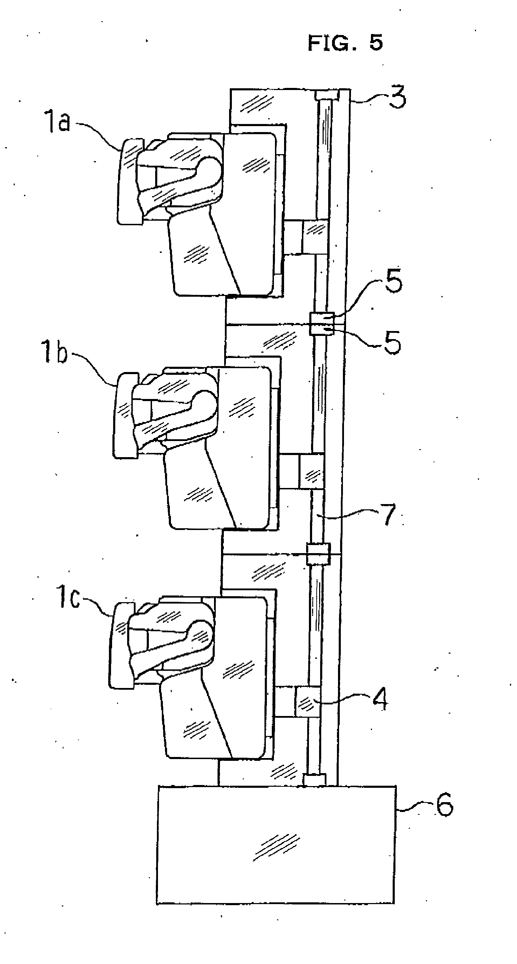

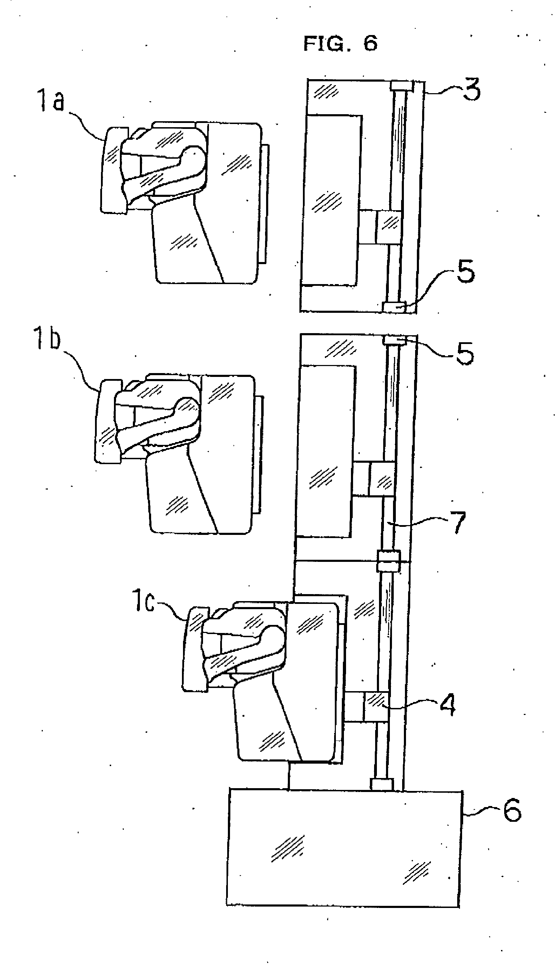

In a liquid infusion apparatus according present invention, three liquid infusion devices 1a to 1c are used, for example, and a common connector 2 of liquid infusion device side for an electric power supply line and a communication line 7 is provided on a bottom surface of each of the liquid infusion devices 1a to 1c, as shown in FIG. 4 to FIG. 6. Further, each of the liquid infusion devices 1a to 1c is mounted detachably on each of racks 3, and a common connector 4 of rack side to be connected to the connector 2 is installed on each of the racks 3. The racks 3 are stacked vertically one another on a base unit 6. Connectors 5 are provided on upper and lower surfaces of each of the racks 3, so that the connector 5 provided on the upper surface of the lower rack 3 is connected electrically with the connector 5 provided on the lower surface of the upper rack 3 stacked on the lower rack 3, A connector (not shown) of base unit side is provided on the base unit 6 so as to be connected to the connector 5 of the lower surface of the lowest rack 3, so that each of the common connectors 4 of rack side is connected with each of the common connectors 2 of liquid infusion device side through each of the electric power supply lines and the communication lines 7, when each of the liquid infusion devices 1a to 1c is connected to each of the respective racks 3.

According to a control system of the liquid infusion apparatus of the present invention, a warning signal is outputted, when such an information that the liquid infusion device 1a, for example, is in the warning signal generating state is sent to a network (not shown). The warning signal generating state of the liquid infusion device 1a is detected through the network by the other liquid infusion devices 1b and 1c and the base unit 6.

Further, the liquid infusion will be carried out by the liquid infusion device 1b, for example, instead of the liquid infusion device 1a, when the liquid infusion from the liquid infusion device 1a is stopped by the warning information. That is, the liquid infusion is started by any alternate liquid infusion device, when the stop of the liquid infusion is detected through the network.

According to the present invention, an additional wiring is not required because the wiring of the electric power supply line and the communication line can be completed by only the racks to which the liquid infusion devices are installed, respectively, are stacked to one another.

The warning signal is generated not only by the liquid infusion device, of which liquid infusion is stopped, but also by the other liquid infusion devices and the base unit, so that such a possibility of the dangerous that the user cannot aware the warning signal generating state due to the malfunction of the warning signal generating device can be reduced. That is, such a state that no liquid medicine is injected into the body of the patient can be prevented.

According to the .control system for the liquid infusion apparatus of the present invention, the wiring of the electric power supply line and the communication line to the liquid infusion devices can be carried out simply and easily, because a network is used as a communication system, a rack structure wherein a plurality of racks each having each of the liquid infusion devices are stacked one another so that the wiring of communication lines and electric power supply lines between the racks are carried out is used, and the connection of the racks and the liquid infusion apparatus is made by only one connector.

Further, such a possibility that the user cannot aware the warning signal generating state of the liquid infusion device because no warning signal is generated due to the malfunction of the warning signal generating device thereof can be obviated. Furthermore, when a warning signal is generated in any of the liquid infusion devices, an information of the warning signal generating state is sent to the network, so that the other liquid infusion devices and the base unit can generate the warning signal and set the liquid infusion apparatus which can start the liquid infusion.

As stated above, according to the present invention, when any liquid infusion devices is stopped the liquid infusion, the liquid infusion is carried out by the other liquid infusion device.

While the invention has been particularly shown and described with reference to the preferred embodiments thereof, it will be understood by those skilled in the art that various changes in form and details may be made therein without departing from the spirit and scope of the invention as defined by the appended claims.

## Claims

1. A control system for a liquid infusion apparatus comprising a plurality of liquid infusion devices, each having a common connector of liquid infusion device side for an electric power supply line and a communication line, a plurality of racks stacked one another, each for receiving each of the liquid infusion devices, a plurality of common connectors of rack side, each mounted on each of the racks so as to be connected to each of the common connectors of liquid infusion device side, when each of the liquid infusion devices is inserted into each of the respective racks, a base unit, and a connector provided on the base unit so as to connect an electric power supply line and a communication line to the connectors of lack side.

2. The control system of the liquid infusion apparatus as claimed in claim 1, wherein a network is used for the communication line, and a warning signal is transmitted from any of the liquid infusion devices through the network to the other liquid infusion devices.

3. The control system of the liquid infusion apparatus as claimed in claim 1 or 2, wherein when the warning signal is generated in any of the liquid infusion devices in the network and the liquid infusion is stopped, the liquid infusion is carried out by the other liquid infusion device in the network.
